# EUROPEAN PATENT APPLICATION

(11) **EP 4 679 316 A1**
(43) Date of publication of application: **14.01.2026**
(21) Application number: 24187430.4
(22) Date of filing: 09.07.2024
(51) Int. Cl.: G06K 7/10, A61B 90/90, A61B 90/98, G06K 17/00, G06Q 10/0833, G06Q 50/04, G16H 20/17

(54) **SYSTEM AND METHOD FOR RFID-BASED LOCALIZATION OF MEDICAL INJECTION DEVICES IN A NEST USING MACHINE LEARNING MODELING**

(71) Applicant: Becton, Dickinson and Company, Franklin Lakes, NJ 07417-1880 (US)
(72) Inventor: GARREC, Ronan, 38640 CLAIX (FR); LUXEMBOURG, David, 38000 Grenoble (FR); MESSAOUD, Mourad, 38000 Grenoble (FR); PERRODIN, Jérémie, Franklin Lakes, NJ 07417 (US)
(74) Representative: Germain Maureau

(57) **Abstract**

Provided herein is a system for localizing RFID-tagged medical injection devices in a nest. The system includes a conveyor that conveys nests along a conveying path, each of the nests retaining RFID-tagged medical injection devices. RFID coupling elements and a RFID reader are positioned at a reading location along the conveying path, to read the RFID-tagged medical injection devices via a mass reading and to read the RFID-tagged nest when passing the reading location, including reading identifiers associated with the devices and the nest, as well as read-related parameters of the devices. A ML model receives a model input from the mass reading comprising the UDIs and read-related parameters of the device RFID tags and determines a location of each of the RFID-tagged medical injection devices within the nest based on analysis of the read-related parameters by the ML model.

## Description

### BACKGROUND OF THE INVENTION

### Field of the Invention

The present disclosure relates generally to RFID-tagged medical injection devices retained in packaging including a nest and, more particularly, to a system and method for localizing RFID-tagged medical injection devices or components thereof within the nest via use of artificial intelligence or machine learning modeling.

### Description of Related Art

Medical injection devices, such as syringes, are used in a variety of environments for administering fluids, such as medications or other drugs, to a patient. Syringes may be provided as prefilled or pre-fillable syringes, which provide the convenience of rapidly delivering the liquid therein to a patient without the need to first aspirate the medication from another container and meter its volume. A syringe will typically include a syringe barrel having a distal end and a proximal end. A needle is connected at the distal end for injecting fluid into the patient and a plunger assembly is inserted through the proximal end that is movable within the barrel to force fluid out from the syringe through the needle.

Components of the syringes as described above often need to be transported from one site to another site during or after manufacturing. For instance, syringe barrels (which, as used herein, may be understood to include the barrel, the needle, and a needle cover) may be transported from a manufacturing site to a site at which the barrels may be filled with a "medicinal fluid," which can refer to a medication or another therapeutic agent used for treatment of chronic or acute conditions, as are known in the art. The filling of the syringe barrels is an automated process that is performed by a fill and finish machine, which dispenses fluid into the syringe barrels in an automated fashion.

During transportation and filling, the syringe barrels may be supported by a holding device, such as a tray or "nest." As known in the art, a nest can be a plate-shaped tray configured to support multiple medical containers in an upright orientation. Nests can comprise multiple "chimneys" aligned according to predetermined rows or a predetermined pattern, with each chimney defining a receptacle or opening configured to receive one medical container. When inserted into and through the receptacle, the medical container is held in the upright orientation in a direction substantially orthogonal to the nest. The nest is usually placed inside a box-shaped tub with an open top, which can be sealed by a sealing cover. The sealing cover can then be removed when the syringes are to be filled with a medicinal fluid by the fill and finish machine.

In an effort to automate the manufacturing of syringes (including filling of the syringes with medicinal fluid) and provide for further traceability of the syringes from the manufacturing process until the final labeling, the final use, or the disposal of the medical devices, it is becoming more common for such syringes to implement RFID tags (including a RFID chip and a RFID antenna) thereon that provide for remote identification and tracing of the syringes. As relates to the manufacturing process, RFID tags may be integrated into the syringes (e.g., in the needle shield thereof) at the site of initial manufacturing, with the tagged syringes (i.e., RFID-tagged syringe barrels) then loaded into a nest and tub for subsequent shipping and filling.

As known in the art, it is desirable to associate a plurality of RFID-tagged syringes with a particular nest in which they are stored. This association may be provided by identifying an RFID tag on the nest and each syringe stored in that nest. For a given nest, the association (so called "aggregation") may be provided as a table with the UDI of the nest and a list of the UDIs of all syringes in that nest - with this table being stored in a database. This aggregation of the UDIs of the syringes with the UDI of the nest is advantageous, as it may simplify subsequent processing/filling of the syringes at a customer location, as the customer may only need to read the tagged nest to determine the UDI of each syringe barrel stored in the nest (rather than reading the UDI of each syringe). The customer may thus be able to efficiently control the fill and finish machine when filling the tagged syringes.

A common method/technique for performing data aggregation for RFID-tagged syringes and nests involves performing of a "mass reading" of the syringes stored in the nest. This mass reading can be performed using a far field RFID antenna and a RFID reader, with all the RFID-tagged syringes in a nest being read collectively, in one reading, and associated with the RFID-tagged nest. However, the data aggregation provided by known mass reading methods is limiting in that - while it does associate the UDIs of the syringes with the UDI of the nest - the mass reading is not able to provide the exact position of each RFID-tagged syringe inside the nest. This inability to register the location of each RFID-tagged syringe inside the nest has a negative impact on processing/workflow efficiency, such as impacting personnel at the location/facility at which filling of the syringe barrels is to be performed. That is, without knowing the location of each RFID-tagged syringe within a nest, controlling of a fill and finish machine for dispensing an appropriate type and quantity of medicinal fluid into each syringe barrel retained within a nest may be inhibited.

Accordingly, a need exists in the art for a system and method by which the location of each RFID-tagged syringe barrel within a nest may be registered. The registering of the location of each RFID-tagged syringe barrel within the nest would be performed with the speed and convenience of existing mass reading techniques, but would also enable accurate location registration for each RFID-tagged syringe barrel within the nest.

### SUMMARY OF THE INVENTION

Provided herein is a system for localizing RFID-tagged medical injection devices in a nest. The system includes a conveyor system configured to convey a plurality of nests along a conveying path, each of the plurality of nests having a nest RFID tag thereon, with each nest retaining a plurality of RFID-tagged medical injection devices therein each having a device RFID tag. The system also includes one or more RFID antennas positioned at a reading location along the conveying path and a RFID reader operably connected with the one or more RFID antennas and configured to perform a mass reading of the device RFID tags and a reading of the nest RFID tag upon a respective nest passing the reading location, wherein reading the nest RFID tag comprises reading a unique device identifier (UDI) from the nest RFID tag and reading the device RFID tags comprises reading a UDI for each of the plurality of RFID-tagged medical injection devices, along with read-related parameters of the device RFID tags. The system further includes at least one processor coupled to a memory and comprising a machine learning (ML) model therein, with the ML model configured to receive a model input from the RFID reader comprising the UDIs of the device RFID tags and the read-related parameters of the device RFID tags and determine a location of each of the plurality of RFID-tagged medical injection devices within the respective nest based on analysis of the read-related parameters of the device RFID tags by the ML model, with the location of each of the plurality of RFID-tagged medical injection devices within the respective nest associated with the UDI for that RFID-tagged medical injection device.

In accordance with some aspects of the disclosure, the read-related parameters of each device RFID tag comprise a received signal strength indicator (RSSI) corresponding to a power level of the RFID tag's backscattered response signal, a read rate (RR) of the device RFID tag; and a response time (T) of the device RFID tag comprising an amount of time needed for the device RFID tag to respond the first time.

In accordance with some aspects of the disclosure, the system further comprises a secondary conveyor configured to individually convey the plurality of RFID-tagged medical injection devices along a secondary conveying path, prior to positioning of the plurality of RFID-tagged medical injection devices in a respective nest, and a sensitivity testing device configured to perform a sensitivity testing of the device RFID tag of each of the plurality of RFID-tagged medical injection devices upon a respective RFID-tagged medical injection device passing a testing location along the secondary conveyor, to determine a tag sensitivity of the device RFID tag, which is a minimum power needed to read the device RFID tag at a given frequency.

In accordance with some aspects of the disclosure, the ML model is configured to: receive another model input from the sensitivity testing device comprising the tag sensitivity of the device RFID tags; and determine a location of each of the plurality of RFID-tagged medical injection devices within the respective nest based on analysis of the read-related parameters and tag sensitivity of the device RFID tags by the ML model, with the location of each of the plurality of RFID-tagged medical injection devices within the respective nest associated with the UDI for that RFID-tagged medical injection device.

In accordance with some aspects of the disclosure, the ML model is configured to analyze the model input in view of a number of nest samples with sample RFID-tagged medical injection devices stored therein, via which the ML model is trained and, for each nest sample, a position of each of the sample RFID-tagged medical injection devices within the nest is known and is associated with read-related parameters of a device RFID tag on each of the sample RFID-tagged medical injection devices.

In accordance with some aspects of the disclosure, each nest sample has a configuration associated therewith, the configuration comprising an arrangement of one or more antennas of a sample RFID reader used to acquire the read-related parameters of the device RFID tag on each of the sample RFID-tagged medical injection devices.

In accordance with some aspects of the disclosure, the ML model is trained with four or more configurations for the nest samples.

In accordance with some aspects of the disclosure, the number of nest samples is 1,000 or more.

In accordance with some aspects of the disclosure, the at least one processor is programmed to receive from the RFID reader, the UDI of the nest RFID tag, and associate the UDI of the nest RFID tag with the UDIs of the device RFID tags, so as to provide an aggregated dataset comprising the UDI of the nest RFID tag, the UDIs of the device RFID tags, and the location of each of the plurality of RFID-tagged medical injection devices within the nest.

In accordance with some aspects of the disclosure, each nest comprises a support plate and a plurality of chimneys extending upwardly from the support plate, each of the plurality of chimneys configured to retain a RFID-tagged medical injection device therein, the plurality of chimneys arranged in a plurality of columns, with each of the plurality of columns comprising a plurality of seats.

In accordance with some aspects of the disclosure, determining the location of each of the plurality of RFID-tagged medical injection devices comprises registering the column and seat of each of the plurality of RFID-tagged medical injection devices.

In accordance with some aspects of the disclosure, the one or more RFID antennas comprises at least a first RFID antenna and a second RFID antenna.

In accordance with some aspects of the disclosure, the system further includes at least one trigger sensor configured to detect when a respective nest is at the reading location, and wherein for each nest on the conveyor system, the at least one sensor is configured to transmit a trigger signal to the RFID reader to cause the RFID reader to perform the mass reading of the device RFID tags and read the nest RFID tag in response to detecting that the nest is at the reading location.

In accordance with some aspects of the disclosure, the plurality of RFID-tagged medical injection devices comprise a plurality of syringe barrels.

In accordance with some aspects of the disclosure, each of the plurality of nests is retained within a respective tub, with the tubs being conveyed along the conveying path.

Also provided herein is a method for localizing RFID-tagged medical injection devices in a nest. The method includes conveying each of a plurality of nests, via a conveyor system, along a conveying path, with each of the plurality of nests having a nest RFID tag thereon, the nest retaining a plurality of RFID-tagged medical injection devices therein each having a device RFID tag. The method also includes providing a RFID reading system at a position along the conveying path, the RFID reading system comprising one or more RFID antennas positioned at a reading location along the conveying path and a RFID reader operably connected with the one or more RFID antennas. The method further includes performing a mass reading of the device RFID tag on each of the plurality of RFID-tagged medical injection devices via the RFID reading system within a respective nest, as the respective nest passes the reading location, with the mass reading retrieving a unique device identifier (UDI) for each of the device RFID tags, along with read-related parameters of the device RFID tags. The method further includes performing a reading of the nest RFID tag of a respective nest via the RFID reading system, as the respective nest passes the reading location, to retrieve a nest UDI from the nest RFID tag and, based on the reading of each of the device RFID tags, determining, via an artificial intelligence (AI) model provided on at least one processor, a location of each of the plurality of RFID-tagged medical injection devices within their respective nest. In determining the location of each of the plurality of RFID-tagged medical injection devices, the method comprises receiving a model input from the RFID reader comprising the UDIs of the device RFID tags and the read-related parameters of the device RFID tags and determining the location of each of the plurality of RFID-tagged medical injection devices within the respective nest based on analysis of the read-related parameters of the device RFID tags by the ML model, with the location of each of the plurality of RFID-tagged medical injection devices within the respective nest associated with the UDI for that RFID-tagged medical injection device.

In accordance with some aspects of the disclosure, the read-related parameters of each device RFID tag comprise a received signal strength indicator (RSSI) corresponding to a power level of the RFID tag's backscattered response signal, a read rate (RR) of the device RFID tag, and a response time (T) of the device RFID tag comprising an amount of time needed for the device RFID tag to respond the first time.

In accordance with some aspects of the disclosure, the method further includes performing a sensitivity testing of the device RFID tag of each of the plurality of RFID-tagged medical injection devices, prior to the mass reading, and via a sensitivity testing device, to determine a tag sensitivity of the device RFID tag, which is a minimum power needed to read the device RFID tag at a given frequency and, in determining the location of each of the plurality of RFID-tagged medical injection devices receiving another model input from the sensitivity testing device comprising the tag sensitivity of the device RFID tags and determining the location of each of the plurality of RFID-tagged medical injection devices within the respective nest based on analysis of the read-related parameters and tag sensitivity of the device RFID tags by the ML model, with the location of each of the plurality of RFID-tagged medical injection devices within the respective nest associated with the UDI for that RFID-tagged medical injection device.

In accordance with some aspects of the disclosure, the method further includes causing the ML model to analyze the model input in view of a number of nest samples with sample RFID-tagged medical injection devices stored therein, via which the ML model is trained, wherein for each nest sample, a position of each of the sample RFID-tagged medical injection devices within the nest is known and is associated with read-related parameters of a device RFID tag on each of the sample RFID-tagged medical injection devices.

In accordance with some aspects of the disclosure, each nest comprises a plurality of chimneys each configured to retain a RFID-tagged medical injection device therein, the plurality of chimneys arranged in a plurality of columns, with each of the plurality of columns comprising a plurality of seats, and wherein determining the location of each of the plurality of RFID-tagged medical injection devices comprises registering the column and seat of each of the plurality of RFID-tagged medical injection devices.

### BRIEF DESCRIPTION OF THE DRAWINGS

FIG. 1A is a perspective view of a RFID-tagged syringe, with which embodiments of the disclosure may be implemented;
FIG. 1B is an exploded view of the syringe of FIG. 1A;
FIG. 1C is a side view of a needle shield included in the syringe of FIG. 1A;
FIG. 2 is a perspective view of packaging, including a tub and nest, for storing and shipping a plurality of medical components, such as the syringe of FIGS. 1A and 1B, with which embodiments of the disclosure may be implemented;
FIG. 3 is a top view of the nest of FIG. 2;
FIG. 4 illustrates an environment in which a method of reading a plurality of RFID-tagged syringe barrels and associated nests may be performed, according to a non-limiting embodiment described herein;
FIG. 5 is a diagram of non-limiting embodiments or aspects of components of one or more devices and/or one or more systems of FIG. 4;
FIG. 6 illustrates an environment in which a sensitivity testing of the RFID tags of a plurality of syringe barrels may be performed, according to a non-limiting embodiment described herein; and
FIG. 7 is a flowchart of a process for localizing RFID-tagged medical injection devices in a nest, according to a non-limiting embodiment described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

As used herein, the terms "communication" and "communicate" may refer to the reception, receipt, transmission, transfer, provision, and/or the like of information (e.g., data, signals, messages, instructions, commands, and/or the like). For one unit (e.g., a device, a system, a component of a device or system, combinations thereof, and/or the like) to be in communication with another unit means that the one unit is able to directly or indirectly receive information from and/or transmit information to the other unit. This may refer to a direct or indirect connection that is wired and/or wireless in nature. Additionally, two units may be in communication with each other even though the information transmitted may be modified, processed, relayed, and/or routed between the first and second unit. For example, a first unit may be in communication with a second unit even though the first unit passively receives information and does not actively transmit information to the second unit. As another example, a first unit may be in communication with a second unit if at least one intermediary unit (e.g., a third unit located between the first unit and the second unit) processes information received from the first unit and communicates the processed information to the second unit. In some non-limiting embodiments or aspects, a message may refer to a network packet (e.g., a data packet and/or the like) that includes data. It will be appreciated that numerous other arrangements are possible.

As used herein, the term "computing device" may refer to one or more electronic devices that are configured to directly or indirectly communicate with or over one or more networks. A computing device may be a mobile or portable computing device, a desktop computer, a server, and/or the like that is specifically configured to provide one or more of the features and/or functions described herein. Furthermore, the term "computer" may refer to a specifically configured computing device that includes the necessary components to receive, process, and output data according to aspects described herein, and normally includes a display, a processor, a memory, an input device, and a network interface. A "computing system" may include one or more computing devices or computers. An "application" or "application program interface" (API) refers to computer code or other data sorted on a computer-readable medium that may be executed by a processor to facilitate the interaction between software components, such as a client-side front-end and/or server-side back-end for receiving data from the client. An "interface" refers to a generated display, such as one or more graphical user interfaces (GUIs) with which a user may interact, either directly or indirectly (e.g., through a keyboard, mouse, touchscreen, etc.). Further, multiple computers, e.g., servers, or other computerized devices directly or indirectly communicating in the network environment may constitute a "system" or a "computing system".

As used herein, the term "communication network" may refer to one or more wired and/or wireless networks. For example, a communication network may include a cellular network (e.g., a long-term evolution (LTE) network, a third generation (3G) network, a fourth generation (4G) network, a fifth generation (5G) network, a code division multiple access (CDMA) network, etc.), a public land mobile network (PLMN), a local area network (LAN), a wide area network (WAN), a metropolitan area network (MAN), a telephone network (e.g., the public switched telephone network (PSTN)), a private network, an ad hoc network, an intranet, the Internet, a fiber optic-based network, a cloud computing network, and/or the like, and/or a combination of these or other types of networks.

It will be apparent that systems and/or methods, described herein, can be implemented in different forms of hardware, firmware, or a combination of hardware and software. The actual specialized control hardware or software code used to implement these systems and/or methods is not limiting of the implementations. Thus, the operation and behavior of the systems and/or methods are described herein without reference to specific software code, it being understood that software and hardware can be designed to implement the systems and/or methods based on the description herein.

No aspect, component, element, structure, act, step, function, instruction, and/or the like used herein should be construed as critical or essential unless explicitly described as such. Also, as used herein, the articles "a" and "an" are intended to include one or more items, and may be used interchangeably with "one or more" and "at least one." Furthermore, as used herein, the term "set" is intended to include one or more items (e.g., related items, unrelated items, a combination of related and unrelated items, etc.) and may be used interchangeably with "one or more" or "at least one." Where only one item is intended, the term "one" or similar language is used. Also, as used herein, the terms "has," "have," "having," or the like are intended to be open-ended terms. Further, the phrase "based on" is intended to mean "based at least partially on" unless explicitly stated otherwise.

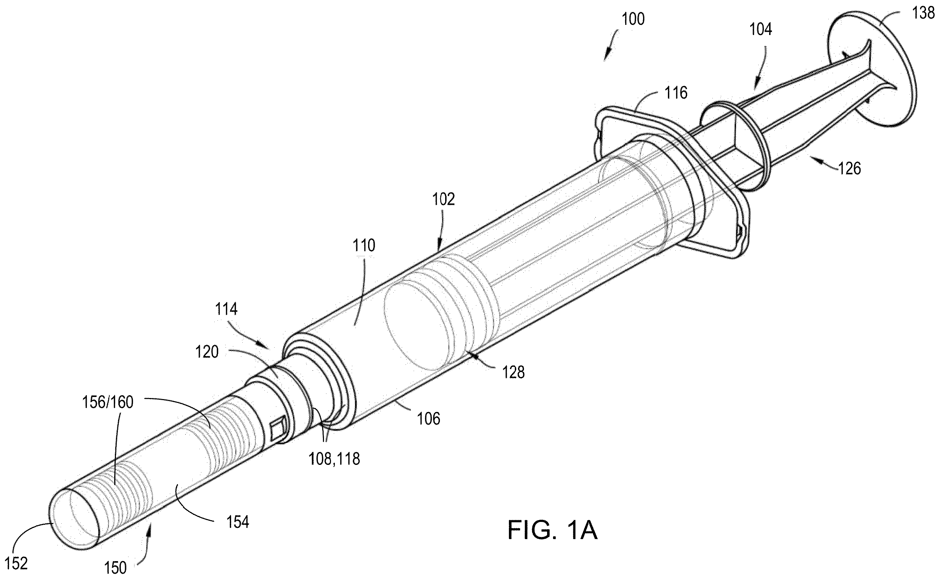

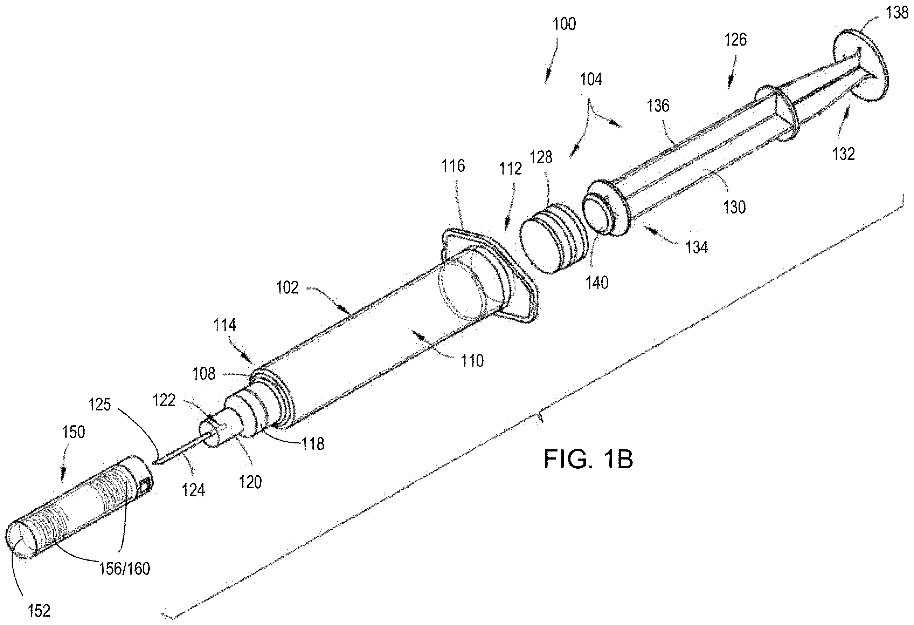

Referring to FIGS. 1A and 1B, shown is a non-limiting embodiment of a RFID-tagged medical injection device 10 with which aspects or embodiments of the disclosure may be implemented. While the medical device is shown and described hereafter as a syringe ("syringe 100"), it is recognized that other RFID-tagged medical devices, including other medical injection devices (e.g., auto-injectors) may be utilized with the system and method of the disclosure described in detail here below.

As shown in FIGS. 1A and 1B, the syringe 100 generally includes a syringe barrel assembly 102 (hereafter "syringe barrel 102") and a plunger assembly 104. The plunger assembly 104 is movable within the syringe barrel 102 along a longitudinal axis to an advanced position to facilitate administering of an injectable fluid (e.g., medication) to a patient, for example. The syringe barrel 102 is formed of a generally cylindrical outer wall 106 and an end member 108 that collectively define a chamber 110 for retaining fluid therein. The syringe barrel 102 includes an open proximal end 112 configured to receive the plunger assembly 104 therein and a distal end 114 at which end member 108 is positioned. The proximal end 112 of the syringe barrel 102 may include a flange 116 to facilitate handling and positioning of the syringe 100 and to maintain the relative position of the syringe barrel 102 with respect to the plunger assembly 104 during medication administration. At the distal end 114, the end member 108 may include a shoulder 118 which narrows with respect to the cylindrical outer wall 106, as well as a hub portion 120 extending out distally from shoulder 118. The hub portion 120 is formed as a partially hollow member that defines a channel 122 therethrough in fluid communication with the chamber 110. A needle 124 is attached to the hub portion 120 within channel 122, such as by being glued or otherwise secured to the hub portion 120, with the needle 124 extending distally from the hub 120 out to a distal needle tip 125.

The plunger assembly 104 of syringe 100 is formed of an elongate plunger rod 126 (more generally "plunger 126," as used hereafter) and a plunger head or stopper 128. The plunger 126 may include a main body 130 extending between a plunger proximal end 132 and a plunger distal end 134. In some embodiments, the main body 130 may include a plurality of elongate vanes or walls 136 extending axially along a length thereof between the plunger proximal end 132 and the plunger distal end 134. A thumb press 138 is positioned at the plunger proximal end 132 that may be engaged by a thumb (or other finger) of the user to apply a distally directed force to the plunger assembly 104 to move the plunger 126 with respect to the syringe barrel 102. In some embodiments, a flanged extension member 140 (e.g., disc-shaped flange) is positioned at the plunger distal end 134 that is configured to mate with the stopper 128. In other embodiments, the plunger distal end 134 may include a female receptacle formed therein that is configured to receive and connect to a protrusion (e.g., pin) extending out proximally from the stopper, with the protrusion and receptacle engaging via threading, for example.

As shown in FIGS. 1A and 1B, a needle shield 150 of syringe barrel 102 (i.e., of the syringe barrel assembly may be coupled to the hub portion 120 and/or shoulder 118 to protect the needle 124. According to aspects of the disclosure, the needle shield 150 may be composed of a rigid outer casing 152 that provides structure to the needle shield 150 and a compliant inner casing or "jupe" 154 that surrounds the needle 124 (when needle shield 150 is coupled to syringe barrel 102). In various embodiments, the outer casing 152 may be made of a rigid material such as polypropylene (PP), polyethylene (PE), polyethylene terephthalate (PET), polystyrene (PS) or polycarbonate (PC), while the inner casing 154 is made of a deformable material such as rubber (e.g., butyl rubber, isoprene rubber, latex rubber, silicone rubber) or a thermoplastic elastomer (TPE), as non-limiting examples.

According to aspects and embodiments of the disclosure, the needle shield 150 may include an RFID tag 156 integrated therein that allows for identification and/or tracking of the syringe 100. That is, the RFID tag 156 may include or store information thereon regarding the contents of the syringe 100 (i.e., the medication or drug included therein, if a prefilled syringe) and the manufacturing history of the syringe and/or may provide location information to an associated reader, so as to enable tracking of the syringe 100. Accordingly, the RFID tag 156 is considered to generally include thereon an electronic product code (EPC) that is specific to the syringe 100. According to aspects or embodiments of the disclosure, the RFID tag 156 may be integrated into the needle shield 150, such as by being inlaid or otherwise disposed between the outer casing 152 and the inner casing 154 of the needle shield 150. According to other embodiments, the RFID tag 156 may be applied to an outer surface of the needle shield 150. In each of these embodiments, the RFID tag 156 conforms to the surface(s) or wall(s) to or within which it is disposed, such that when the RFID tag 156 is integrated with/on a cylindrical component such as the needle shield 150, the RFID tag 156 will have a curvature that matches that of the needle shield 150.

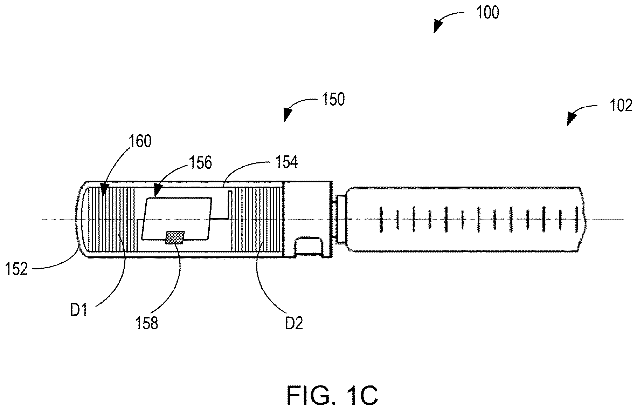

According to embodiments, and as shown in FIG. 1C, the RFID tag 156 includes an RFID chip 158 connected to an RFID dipole coupling element or antenna 160. The antenna 160 of RFID tag 156 may include two legs or dipole elements D1, D2, with the RFID chip 158 disposed between extremities of the poles D1, D2, such as being centered therebetween. In some embodiments, the RFID tag 156 may be positioned and oriented within needle shield 150 such that a dipole axis, A_{D}, of the RFID tag 156 is approximately aligned with a longitudinal axis, Ac, of the needle shield 150 (and a longitudinal axis, As, of the syringe 100), such that more strict quality controls may be implemented during manufacturing/assembly of the syringe 100 and the cap 150 thereof.

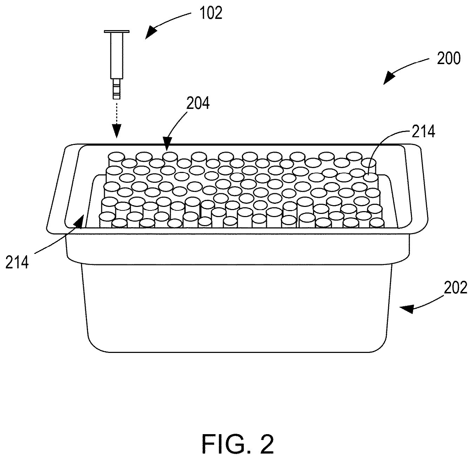

Referring to FIG. 2, shown is a non-limiting embodiment of packaging 200 that may be used for the packaging and transporting of a plurality of medical components therein, such as syringes 100 or syringe barrel assemblies 102 thereof shown in FIGS. 1A-1C. The packaging 200 includes a container or tub 202 in which a nest 204 may be housed, with the nest 204 configured to retain a plurality of syringes 100 or syringe barrels 102 therein. The packaging 200 may also include a sealing cover (not shown) that may be applied over the tub 202 after the nest 204 is packaged therein, to provide a sterile environment for storing and shipping the syringes 100 or syringe barrels 102.

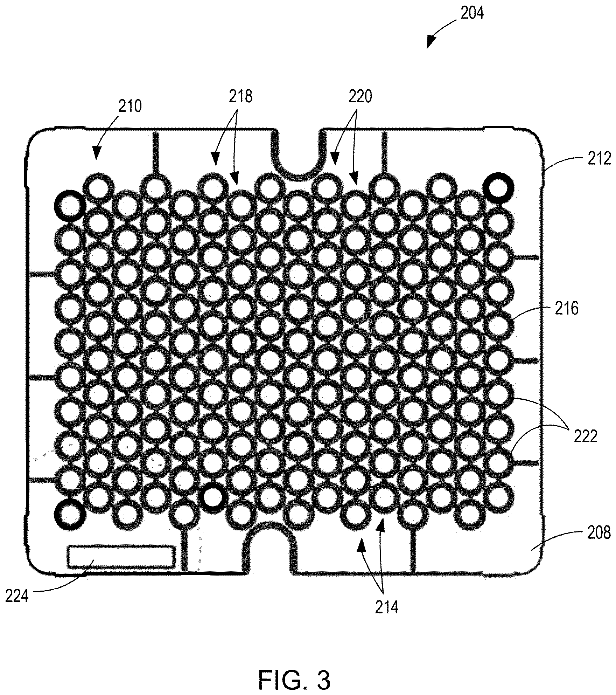

As shown in FIG. 2 and also in FIG. 3, an exemplary nest 204 that may be retained in tub 202 comprises a support plate 208 having a first or upper surface 210, a second or lower surface (now shown), and a peripheral edge 212 extending about the support plate 208. In some examples, the perimeter of support plate 208 is substantially rectangular in shape, while in other embodiments, the support plate 208 may be square. The nest 204 furthers comprise tubular walls, members, or ridges - referred to hereafter as "chimneys 214" - that protrude outward and upward from a plane defined by the upper surface 210 of the support plate 208. Each chimney 214 can include a cylindrical wall 216 that defines a receptacle 218 configured to receive a syringe barrel 102 therein. The receptacle 218 includes a top opening at a top end of the chimney 214 and a bottom opening at a bottom end of the chimney 214, with the receptacle sized to receive the cylindrical outer wall 106 of barrel 102 therein and the flange 116 of barrel 102 resting against an upper edge of the chimney 214. As shown best in FIG. 3, according to some aspects of the disclosure, the nest 204 can be configured to include one-hundred sixty (160) chimneys 214 for receiving syringe barrels 102, although it is recognized that another number of chimneys 214 may be provided on nest 204 that is dependent on the size of the syringe barrels 102 retained therein (e.g., 1-3 mL syringe barrels, 5 mL syringe barrels, 10 mL syringe barrels, etc.). The chimneys 214 may be considered as being arranged in a plurality of columns 220 (e.g., 16 columns, in the embodiment of FIG. 3), with each of the columns 220 including a plurality of "seats" 222 that correspond to the individual chimneys 214 (e.g., 10 seats in each column, with 160 seats total, in the embodiment of FIGS. 3A and 3B).

According to aspects of the disclosure, nest 204 includes an RFID tag 224 thereon, such as on the upper surface 210 of support plate 208, adjacent a corner thereof. The RFID tag 224 allows for identification of the nest 204. That is, the RFID tag 224 may include or store thereon a unique device identifier (UDI) that is specific to the nest 204, with it recognized that each specific nest 204 will have associated therewith the particular RFID-tagged syringe barrels 102 that are retained in the nest 204 and the location of each syringe barrel within the nest 204, so as to enable localizing of the syringe barrels 102 within nest 204. The RFID tag identifier of the nest 204 and of the RFID-tagged syringe barrels 102 retained thereby may be used for registration purposes and dispensing of a medication or drug into the syringe barrels 102 (if a prefilled syringe) during a fill and finish process, as explained in further detail below.

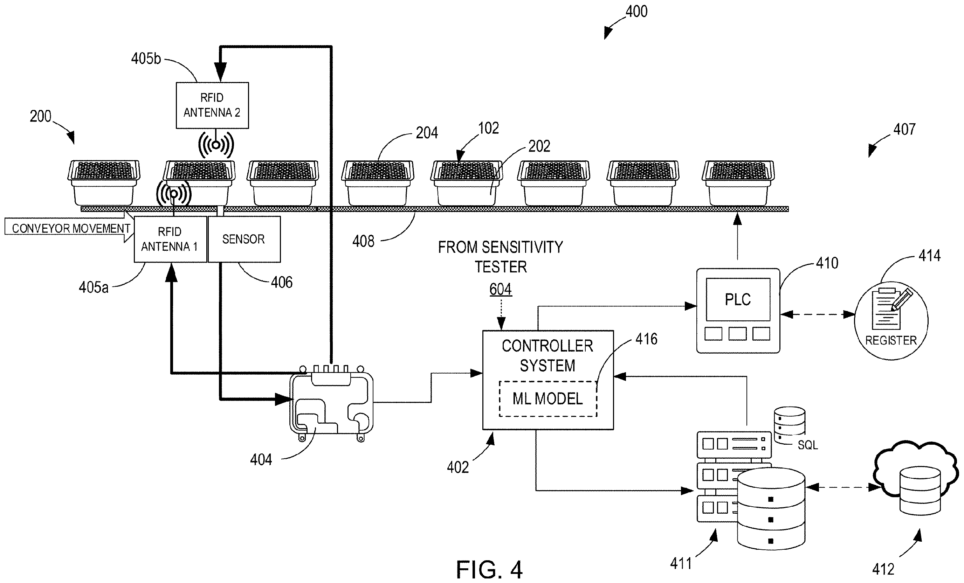

Referring now to FIG. 4, a manufacturing and reading environment or system 400 is illustrated within which or by which RFID-tagged syringe barrels 102 and an associated RFID-tagged nest of a packaging (such as the packaging 200 of FIG. 2) may be read, in accordance with an aspect of the disclosure. As shown in FIG. 4, a plurality of packagings 200 - each including a plurality of RFID-tagged syringe barrels 102, nest 204, and tub 202 - may be provided to system 400. According to aspects of the disclosure, system 400 operates to perform a mass reading of all RFID-tagged syringe barrels 102 retained within a respective RFID-tagged nest 204 as part of a process for localizing the RFID-tagged syringe barrels 102 retained within that nest 204, including identifying the specific location (i.e., chimney 214) of each RFID-tagged syringe barrel 102 within the nest 204. As indicated above, "syringe barrels 102" may be understood to refer to an assembly of the cylindrical outer wall 106, end member 108, shoulder 118, hub portion 120, needle 124, and needle shield 150 (including RFID tag 156). In localizing each RFID-tagged syringe barrel 102 within a nest 204, the system 400 makes use of an artificial intelligence (AI) or machine learning (ML) model (hereafter, generally "ML model") that compares information acquired from the mass reading (and, optionally, from a separate sensitivity testing, as explained in further detail below) with data stored in the model, in order to determine the specific location of each RFID-tagged syringe barrel 102 within the nest 204. As will be explained in further detail below, the information acquired for each syringe barrel 102 from the mass reading (and the information stored in the model) may comprise "read-related parameters" or "reading -related parameters" will include:
- the average received signal strength indicator (RSSI), which corresponds to the power level of the RFID tag's backscattered response signal;
- the RFID tag's read rate (RR);
- the response time (T) of each RFID tag (i.e., the amount of time needed for each tag to respond the first time); and

Also, as indicated above, information acquired for each syringe barrel 102 may optionally include the RFID tag's sensitivity, which is the minimum power needed to read a tag at a given frequency.

As shown in FIG. 4 system 400 may include a controller system 402, a RFID reader 404, RFID antennas 405 including at least a first RFID antenna 405a and a second RFID antenna 405b (collectively a "RFID reading system"), a trigger sensor 406, a conveyor system 407 - including a conveyor 408 and programmable logic controller (PLC) 410 - a local database system 411, and/or an external database system 412.

Controller system 402 may include one or more devices capable of receiving information and/or data from RFID reader 404, trigger sensor 406, conveyor system 407, local database system 411, external database system 412 and/or a sensitivity testing device 604 (e.g., via a communication network, via a direct wired and/or wireless connection, etc.), and/or communicating information and/or data to RFID reader 404, trigger sensor 406, conveyor system 407, local database system 411, external database system 412, and/or sensitivity testing device 604 (e.g., via communication network, via a direct wired and/or wireless connection, etc.). For example, controller system 402 may include a computing device, such as a server, a group of servers, and/or other like devices. In some non-limiting embodiments or aspects, controller system 402 may include middleware, such as Message Queuing Telemetry Transport (MQTT) protocol-based middleware, and/or the like, configured to manage communication with and/or control of operations of RFID reader 404, trigger sensor 406, conveyor system 407 (i.e., PLC 410 of conveyor system 407, etc.), local database system 411, and/or external database system 412.

RFID reader 404 may include one or more devices capable of receiving information and/or data from controller system 402, trigger sensor 406, conveyor system 407, local database system 411 and/or external database system 412 (e.g., via a communication network, via a direct wired and/or wireless connection, etc.) and/or communicating information and/or data to controller system 402, trigger sensor 406, conveyor system 407, local database system 411 and/or external database system 412 (e.g., via communication network, via a direct wired and/or wireless connection, etc.).

RFID reader 404 may include a passive RFID reader, an active RFID reader, or any combination thereof. RFID reader 404 may be configured to read - from the plurality of RFID tags 156 on the RFID-tagged syringe barrels 102 and from the RFID tag 224 on each nest 204 (within a tub 202) - identifiers associated with the syringe barrel 102 and the nest 204. For example, RFID reader 404 may be configured to attempt to read the RFID tag 156 on each RFID-tagged syringe barrel 102 and the RFID tag 224 on a nest 204 in response to receiving a trigger signal from trigger sensor 406. In reading the RFID tags 156 on the RFID-tagged syringe barrels 102, the RFID reader 404 may also acquire information for each RFID tag 156 regarding: the average RSSI for each RFID tag, which corresponds to the power level of the RFID tag's backscattered response signal; the read rate(RR) for each RFID tag; and the response time (T) of each RFID tag.

RFID antennas 405a, 405b ) of RFID reader 404 may be located proximate to conveyor 408 such that, as conveyor 408 moves the tubs 202 along the production line, the tubs 202 are moved one-by-one to be adjacent to (and/or to be paused adjacent to for a predetermined period of time) antennas 405a, 405b of RFID reader 404 at a "reading location", and then moved past antennas 405a, 405b of RFID reader 404. While only a first antenna 405a and a second antenna 405b are shown in FIG. 4, it is recognized that system 400 could include only a single antenna 405 or more than two antennas (i.e., antennas 405a, 405b... 405n) are associated with the RFID reader 404. The antennas 405a, 405b may be positioned at various locations along the conveyor 408 at the reading location (e.g., on opposing sides of the conveyor 408) to facilitate a mass reading of the RFID tags 156 of the syringe barrels 102 and reading of the RFID tag 224 on a nest 204 in any of a number of "configurations", with additional positioning options and configurations of antenna(s) being shown in phantom in FIG. 4.

Trigger sensor 406 may include one or more devices capable of receiving information and/or data from controller system 402, RFID reader 404, conveyor system 407, local database system 411 and/or external database system 412 (e.g., via a communication network, via a direct wired and/or wireless connection, etc.) and/or communicating information and/or data to controller system 402, RFID reader 404, conveyor system 407, local database system 411 (e.g., via communication network, via a direct wired and/or wireless connection, etc.).

Trigger sensor 406 may include at least one sensor (e.g., an optical sensor, a laser sensor, etc.) configured to detect or be trigged when an individual tub 202 is in a predetermined location relative to antennas 405a, 405b of the RFID reader 404 (e.g., when a tub 202 is at the reading location, adjacent antennas 405a, 405b). Trigger sensor 406 may be located proximate or adjacent to antennas 405a, 405b of RFID reader 404 and/or conveyor 408 and/or at a position relative to antennas 405a, 405b of RFID reader 404 and/or conveyor 408 at which, when a tub 202 is in the predetermined location relative to antennas 405a, 405b of the RFID reader 404, the tub 202 is within the field of detection or view of trigger sensor 406 such that trigger sensor 406 is triggered or detects the tub 202. In response to being triggered or detecting a tub 202 in the predetermined location relative to antennas 405a, 405b of the RFID reader 404, trigger sensor 406 may be configured to communicate or transmit a trigger signal to RFID reader 404 to trigger or cause RFID reader 404 to attempt to read the RFID tags 156 on syringe barrels 102 and the RFID tag 224 on the nest 204.

Conveyor system 407 may include one or more devices capable of receiving information and/or data from controller system 402, RFID reader 404, trigger sensor 406, local database system 411 and/or external database system 412 (e.g., via a communication network, via a direct wired and/or wireless connection, etc.) and/or communicating information and/or data to controller system 402, RFID reader 404, trigger sensor 406, local database system 411 and/or external database system 412 (e.g., via communication network, via a direct wired and/or wireless connection, etc.).

Conveyor system 407 may include a conveyor 408 (e.g., a motorized belt conveyor, a motorized roller conveyor, a motorized chain conveyor, a motorized trolley conveyor, etc.) that conveys tubs 202 along a path (e.g., linear path). Each of the tubs 202 may be retained on the conveyor 408, with the tubs 202 held in position by mechanical force or another suitable means. Conveyor 408 may be configured to individually move (e.g., move one-by-one, etc.) tubs 202 in the production line adjacent to and past antennas 405a, 405b of RFID reader 404. For example, tubs 202 may be arranged on conveyor 408, and/or conveyor 408 may be configured to move tubs 202 together in a same direction such that tubs 202 are moved one-by-one adjacent to (and/or paused adjacent to for a predetermined period of time), and moved then past, antennas 405a, 405b of RFID reader 404.

The PLC 410 of conveyor system 407 is configured to control one or more operations of conveyor system 407, including conveyor 408. The PLC 410 may be configured to track a sequential order of the tubs 202 in the production line (on conveyor 408) and the location of RFID-tagged syringe barrels 102 in the nests 204 of tubs 202, with such tracking performed via one or more shift registers (e.g., shift register 414) and/or sensors, according to existing manufacturing techniques.

Local database system 411 may include one or more devices capable of receiving information and/or data from controller system 402, RFID reader 404, trigger sensor 406, conveyor system 407, and/or external database system 412 (e.g., via a communication network, via a direct wired and/or wireless connection, etc.) and/or communicating information and/or data to controller system 402, RFID reader 404, trigger sensor 406, conveyor system 407, and/or external database system 412 (e.g., via communication network, via a direct wired and/or wireless connection, etc.). In some non-limiting embodiments or aspects, local database system 411 includes one or more local databases (e.g., local to and/or implemented by controller system 402, etc.). Local database system 411 may be configured to store data on each RFID-tagged nest 204 (i.e., the UDI of each nest RFID tag 224), along with data on the RFID-tagged syringe barrels 102 retained in each nest 204 and the ordering/locations of the RFID-tagged syringe barrels 102 retained in each nest 204, as explained in further detail below. In some embodiments, local database system 411 may retrieve data on each RFID-tagged nest 204 and data on the RFID-tagged syringe barrels 102 retained in each nest 204 from external database system 412, for subsequent storage on local database system 411.

External database system 412 may include one or more devices capable of receiving information and/or data from controller system 402, RFID reader 404, trigger sensor 406, conveyor system 407, and/or local database system 411 (e.g., via a communication network, via a direct wired and/or wireless connection, etc.) and/or communicating information and/or data to controller system 402, RFID reader 404, trigger sensor 406, conveyor system 407, and/or local database system 410 (e.g., via communication network, via a direct wired and/or wireless connection, etc.). In some non-limiting embodiments or aspects, external database system 412 includes one or more external databases (e.g., external to and/or not implemented by controller system 102, etc.). External database system 412 may be configured to store data on each RFID-tagged nest 204 (i.e., the UDI of each nest RFID tag 224), along with data on the RFID-tagged syringe barrels 102 retained in each nest 204 and the ordering/locations of the RFID-tagged syringe barrels 102 retained in each nest 204, as explained in further detail below.

The number and arrangement of devices shown in FIG. 4 is provided as an example. There can be additional devices, fewer devices, different devices, or differently arranged devices than those shown in FIG. 4. Furthermore, two or more devices shown in FIG. 4 can be implemented within a single device, or a single device shown in FIG. 4 can be implemented as multiple, distributed devices. Additionally, or alternatively, a set of devices (e.g., one or more devices, etc.) of system 400 can perform one or more functions described as being performed by another set of devices of system 400.

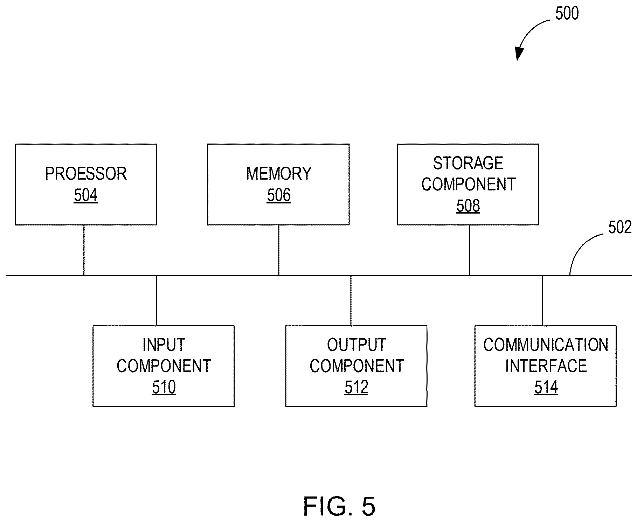

Referring now to FIG. 5, FIG. 5 is a diagram of example components of a device 500. Device 500 may correspond to one or more devices of controller system 402, RFID reader 404 (e.g., one or more devices of a system of RFID reader 404, etc.), trigger sensor 406 (e.g., one or more devices of a system of trigger sensor 406, etc.), one or more devices of conveyor system 407, one or more devices of local database system 411 and/or one or more devices of external database system 412. In some non-limiting embodiments or aspects, one or more devices of controller system 402, RFID reader 404 (e.g., one or more devices of a system of RFID reader 404, etc.), trigger sensor 406 (e.g., one or more devices of a system of trigger sensor 406, etc.), one or more devices of conveyor system 407 one or more devices of local database system 411 and/or one or more devices of external database system 412, may include at least one device 500 and/or at least one component of device 500. As shown in FIG. 5, device 500 may include bus 502, processor 504, memory 506, storage component 508, input component 510, output component 512, and communication interface 514.

Bus 502 may include a component that permits communication among the components of device 500. In some non-limiting embodiments or aspects, processor 504 may be specifically configured to perform one or more of the mixing aspects described in hardware, software, or a combination of hardware and software. For example, processor 504 may include a processor (e.g., a central processing unit (CPU), a graphics processing unit (GPU), an accelerated processing unit (APU), etc.), a microprocessor, a digital signal processor (DSP), and/or similar processing component (e.g., a field-programmable gate array (FPGA), an application-specific integrated circuit (ASIC), etc.) that can be programmed to perform a function. Memory 506 may include random access memory (RAM), read-only memory (ROM), and/or another type of dynamic or static storage device (e.g., flash memory, magnetic memory, optical memory, etc.) that stores information and/or instructions for use by processor 504.

Storage component 508 may store information and/or software related to the operation and use of device 500. For example, storage component 508 may include a hard disk (e.g., a magnetic disk, an optical disk, a magneto-optic disk, a solid state disk, etc.), a compact disc (CD), a digital versatile disc (DVD), a floppy disk, a cartridge, a magnetic tape, and/or another type of computer-readable medium, along with a corresponding drive.

Input component 510 may include a component that permits device 500 to receive information, such as via user input (e.g., a touch screen display, a keyboard, a keypad, a mouse, a button, a switch, a microphone, etc.). Additionally or alternatively, input component 510 may include a sensor for sensing information (e.g., a global positioning system (GPS) component, an accelerometer, a gyroscope, an actuator, etc.). Output component 512 may include a component that provides output information from device 500 (e.g., a display, a speaker, one or more light-emitting diodes (LEDs), etc.).

Communication interface 514 may include a transceiver-like component (e.g., a transceiver, a separate receiver and transmitter, etc.) that enables device 500 to communicate with other devices, such as via a wired connection, a wireless connection, or a combination of wired and wireless connections. Communication interface 514 may permit device 500 to receive information from another device and/or provide information to another device. For example, communication interface 514 may include an Ethernet interface, an optical interface, a coaxial interface, an infrared interface, a radio frequency (RF) interface, a universal serial bus (USB) interface, a Wi-Fi^{®} interface, a cellular network interface, and/or the like.

Device 500 may perform one or more processes described herein. Device 500 may perform these processes based on processor 504 (e.g., a central processing unit (CPU), a graphics processing unit (GPU), etc.) executing software instructions stored by a computer-readable medium, such as memory 506 and/or storage component 508. A computer-readable medium (e.g., a non-transitory computer-readable medium) is defined herein as a non-transitory memory device. A non-transitory memory device includes memory space located inside of a single physical storage device or memory space spread across multiple physical storage devices.

Software instructions may be read into memory 506 and/or storage component 508 from another computer-readable medium or from another device via communication interface 514. When executed, software instructions stored in memory 506 and/or storage component 508 may cause processor 504 to perform one or more processes described herein. Additionally or alternatively, hardwired circuitry may be used in place of or in combination with software instructions to perform one or more processes described herein.

Memory 506 and/or storage component 508 may include data storage or one or more data structures (e.g., a database, etc.). Device 500 may be capable of receiving information from, storing information in, communicating information to, or searching information stored in the data storage or one or more data structures in memory 506 and/or storage component 508.

The number and arrangement of components shown in FIG. 5 are provided as an example. In some non-limiting embodiments or aspects, device 500 may include additional components, fewer components, different components, or differently arranged components than those shown in FIG. 5. Additionally or alternatively, a set of components (e.g., one or more components) of device 500 may perform one or more functions described as being performed by another set of components of device 500.

According to aspects of the disclosure, it is recognized that reading of the RFID tags 156 on syringe barrels 102 and reading of the RFID tag 224 of an associated nest 204 by the RFID reader 404 can be used to localize individual RFID-tagged syringe barrels 102 within each nest 204. Specifically, in performing a mass reading of the RFID tags 156 on syringe barrels 102 (i.e., reading all RFID tags 156 on syringe barrels 102 at the same time), the information acquired/retrieved from the reading can be used to localize individual RFID-tagged syringe barrels 102 within a respective nest 204, by analyzing the acquired/retrieved information in comparison to a trained ML model 416 (see FIG. 4), in order to identify the location of each syringe barrel based on the acquired/retrieved information. The ML model 416 may be programmed into a processor (e.g., processor 504) included in any of a number of components in system 400, such as controller system 402, PLC 410, or local database system 411, or another component separate from such devices/components. For purposed of convenience, ML model 416 is shown as being programmed into controller system 402 (i.e., into a processor 504 of controller system 402), although it is understood that aspects of the disclosure are not limited to such an embodiment.

As indicated above, in reading the RFID tags 156 on the RFID-tagged syringe barrels 102, the RFID reader 404 acquires/retrieves read-related parameters for each RFID tag 156 regarding: the UDI, the RSSI; the RR; and the response time (T). Thus, for a mass reading of the RFID tags 156, a data table will be provided with n lines (corresponding to the number of syringe barrels 102 retained by the nest 204), with each line including columns for the acquired/retrieved parameters (UDI; sensitivity; RSSI; RR; T;)_{1...n}. The mass reading of the RFID tags 156 with these acquired/retrieved parameters for each tag may be compared to a trained ML model 416 that ties values of these parameters to specific locations in a nest, in order to assign locations to each of the RFID-tagged syringe barrels 102 within a nest 204. Description of a training technique for the ML model 416 follows here below.

Initially, it is desired to train the ML model 416 to analyze the above-identified parameters for mass reading of RFID-tagged syringe barrels in a nest, for each of a number of different "configurations" of antennas of an RFID reader. As previously described, such configurations may include a single antenna at a first position, a single antenna at a second position, multiple antennas with a first arrangement, multiple antennas with a second arrangement, etc., with it recognized that training the ML model 416 for at least four (4) different configurations is desired. Reading in k number of configurations (k≥4) allows collection/assembly of a Table with n*k Lines:

**TABLE 1**

| | | | |
|---|---|---|---|
| UDI₁ | RSSI(1,Config1) | RR(1,Config1) | T(1,Config1) |
| UDI₁ | RSSI(1,Config2) | RR(1,Config2) | T(1,Config2) |
| .... | .... | .... | .... |
| UDI₁ | RSSI(1,Configk) | RR(1,Configk) | T(1,Configk) |
| UDI₂ | RSSI(2,Config1) | RR(2,Config1) | T(2,Config1) |
| UDI₂ | RSSI(2,Config2) | RR(2,Config2) | T(2,Config2) |
| .... | .... | .... | .... |
| UDI₂ | RSSI(2,Configk) | RR(2,Configk) | T(2,Configk) |
| .... | .... | .... | .... |
| UDI₁ | RSSI(n,Config1) | RR(n,Config1) | T(n,Config1) |
| UDIₙ | RSSI(n,Config2) | RR(n,Config2) | T(n,Config2) |
| .... | .... | .... | .... |
| UDI₁ | RSSI(n,Configk) | RR(n,Configk) | T(n,Configk) |

As indicated above, it may be desirable to also acquire/determine the sensitivity (i.e., the minimum power needed to read a RFID tag, at a given frequency) of the RFID tag 156 of each syringe barrel 102, for inclusion of this data in the trained ML model 416 and in connection with the parameters acquired/retrieved in the mass reading. With inclusion of the tag sensitivity parameter, the Table of available data is thus a table with n*k lines, with an additional column:

**TABLE 2**

| | | | | |
|---|---|---|---|---|
| UDI₁ | Sensitivity₁ | RSSI(1,Config1) | RR(1,Config1) | T(1,Config1) |
| UDI₁ | Sensitivity₁ | RSSI(1,Config2) | RR(1,Config2) | T(1,Config2) |
| .... | .... | .... | .... | .... |
| UDI₁ | Sensitivity₁ | RSSI(1,Configk) | RR(1,Configk) | T(1,Configk) |
| UDI₂ | Sensitivity₂ | RSSI(2,Config1) | RR(2,Config1) | T(2,Config1) |
| UDI₂ | Sensitivity₂ | RSSI(2,Config2) | RR(2,Config2) | T(2,Config2) |
| .... | .... | .... | .... | .... |
| UDI₂ | Sensitivity₂ | RSSI(2,Configk) | RR(2,Configk) | T(2,Configk) |
| .... | .... | .... | .... | .... |
| .... | .... | .... | .... | .... |
| .... | .... | .... | .... | .... |
| .... | .... | .... | .... | .... |
| UDIₙ | Sensitivityₙ | RSSI(n,Config1) | RR(n,Config1) | T(n,Config1) |
| UDIₙ | Sensitivityₙ | RSSI(n,Config2) | RR(n,Config2) | T(n,Config2) |
| .... | .... | .... | .... | .... |
| UDIₙ | Sensitivityₙ | RSSI(n,Configk) | RR(n,Configk) | T(n,Configk) |

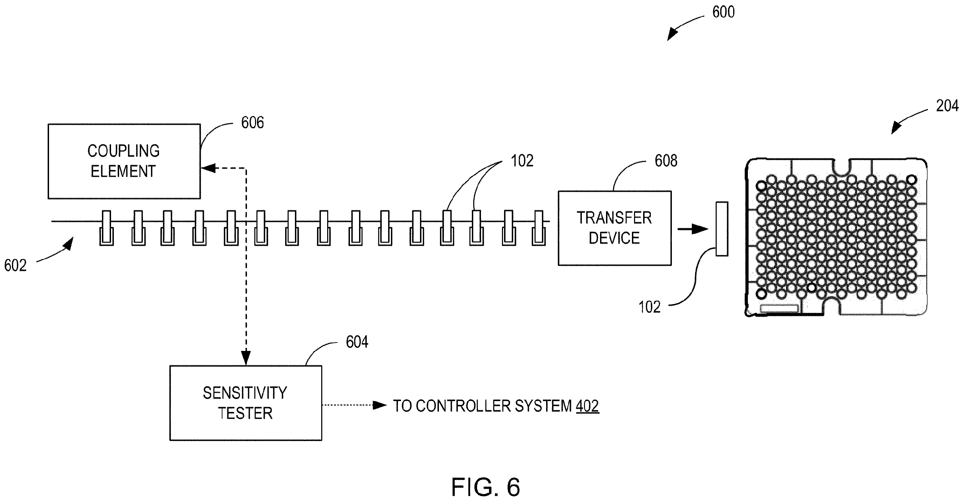

Regarding the acquiring of the sensitivity parameter for the RFID tag 156 of each syringe barrel 102, it is recognized that the sensitivity would be measured by a separate testing device before performing of the mass reading by RFID reader 402. FIG. 6 illustrates an exemplary set-up or system 600 used to perform the sensitivity testing for the RFID tag 156 of each syringe barrel 102, with it recognized that the system 600 would be provided upstream from the system 400 of FIG. 4. That is, as shown in FIG. 6, sensitivity testing is performed individually on each RFID tag 156, prior to the syringe barrel 102 being transferred/stored within a nest 204. The syringe barrels 102 may be processed along an initial or "secondary" conveyor line 602 (i.e., secondary to conveyor 408), with the syringe barrels 102 being conveyed along the line 602 to a testing location, where a testing device 604 (and associated coupling element 606) operates to measure a sensitivity of the RFID tag 156 of a respective syringe barrel 102. In some embodiments, the testing device 604 also reads the RFID tag 156 of each syringe barrel 102 to retrieve the UDI therefrom, such that the UDI and sensitivity reading may be associated with each other for the particular RFID-tagged syringe barrel 102. According to embodiments, the sensitivity data for each RFID-tagged syringe barrel 102, along with the UDI for each RFID-tagged syringe barrel 102, may be transmitted from testing device 604 to system 400 (i.e., to controller system 402, PLC 410, and/or database(s) 411, 412), for inclusion of such data/readings with data/readings acquired during a mass reading performed by system 400, as explained in further detail below.

Upon testing of the RFID tag sensitivity (and retrieval of the tag UDI) for each RFID-tagged syringe barrel 102, the syringe barrel 102 is conveyed further down the conveyor line 602 to a transfer device 608 that functions to transfer the syringe barrel 102 to a nest 204, for preparation of a nest 204 of syringe barrels 102 being further packaged (i.e., within tub 202) and then sent to system 400 for further processing.

With it being understood that the above-described read-related parameters are included in the ML model 416, a supervised learning/training of the ML model 416 may be performed in order to enable the model to predict the position of each RFID-tagged syringe barrel within a nest upon the mass reading of the syringe barrels 102 (i.e., the RFID tags 156 thereof).

In generating/training the ML model 416, a labeled dataset comprising a collection of *N* nests (N>1000) is provided. For each nest, input data/parameters are provided from a mass reading of RFID-tagged syringe barrels in the nest - with the mass reading being performed with the RFID reader (and antenna(s) thereof) in a k configuration. Optionally, and as explained in detail above, the sensitivity of the RFID tag on each syringe barrel is also known (from a previous testing). In addition to having the input data/parameters provided from the mass reading (and from the sensitivity testing), the position of each RFID-tagged syringe barrel within the nest is known and provided to the model - with the position of each RFID-tagged syringe barrel described by the number the row and seat (or line and column) in the nest, e.g., syringe barrel_1 is in position (x₁;y₁).... syringe barrel n is in position (xₙ;yₙ). The position of each RFID-tagged syringe barrel within the nest is then associated with the input data/parameters for each RFID-tagged syringe barrel provided from the mass reading (and from the sensitivity testing).

With a dataset of N nests (N>1000) provided, and with input data/parameters from the mass reading and an associated position of each RFID-tagged syringe barrel within the nest provide for each nest, the ML model 416 may be trained to predict a list of n positions (x₁;y₁)....(xₙ;yₙ) using the table of data collected after mass reading (and, optionally, sensitivity data), for each of the N nests. A Table may thus be provided as:

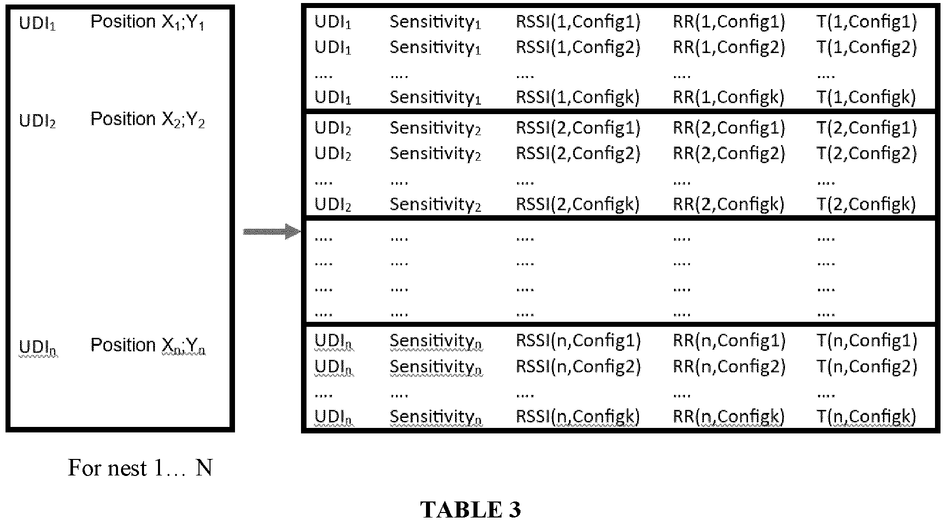

Once the ML model 416 is trained, the position of each syringe barrel in any given nest -characterized by the mass reading station in the *k* configuration (and, optionally, the sensitivity data available) - can be determined according to:

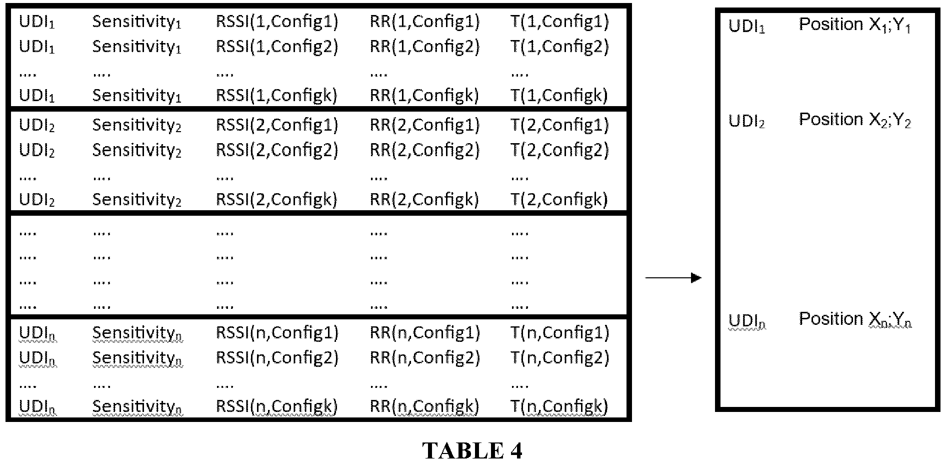

Finally, once the position of each syringe is known, the UDI of the nest 204 can be associated to the list of RFID-tagged syringe barrels 102 to generate the aggregated list:

**TABLE 5**

| | | |
|---|---|---|
| UDINest | UDI₁ | Position X₁;Y₁ |
| | UDI₂ | Position X₂;Y₂ |
| | .... | |
| | UDI₁ | Position Xₙ;Yₙ |

According to embodiments of the disclosure, the ML model 416 may be checked and validated by comparing the prediction of the ML model 416 using specific test samples (i.e., nests) for which positions of the syringe barrels in the nest are known. The validation may be performed for a suitable number of test samples (e.g.,>100 nests) before the ML model 416 is considered validated. Upon validation, the ML model 416 may then be used to predict the position of RFID-tagged syringe barrels 102 within a nest 204 during manufacturing/processing performed on system 400 (and, optionally, system 600), upon mass reading of RFID-tagged syringe barrels 102 within a respective nest 204.

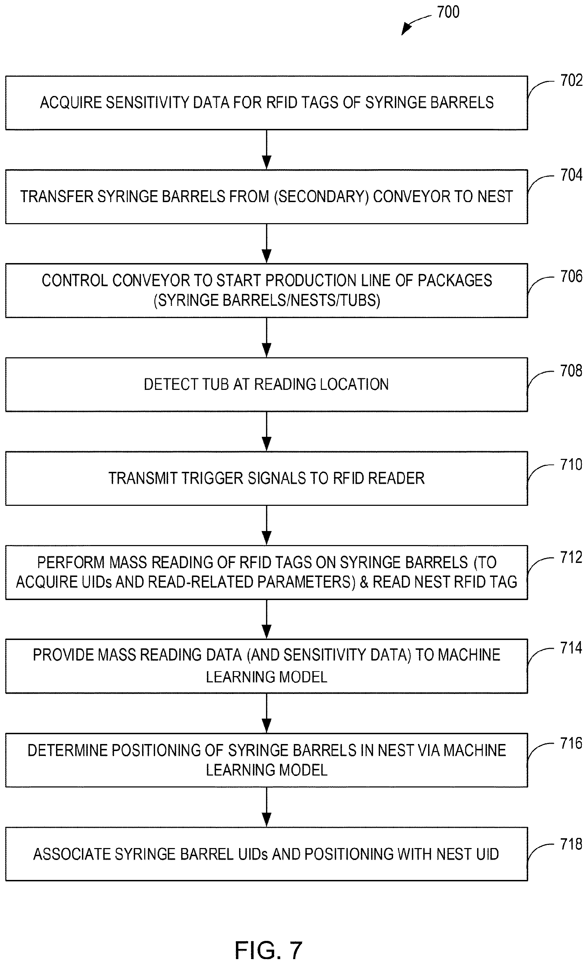

Referring now to FIG. 7, a flowchart is provided for a non-limiting embodiment or aspect of a method 700 for localizing individual RFID-tagged syringe barrels 102 within a nest 204 of a tub 202 that is advanced along a conveyor. Specifically, method 700 reads the RFID tag of each syringe barrel 102 within a nest 204 via a mass reading, with information retrieved from the mass reading being provided to an ML model 416 that functions to determine the location of each of the plurality of RFID-tagged medical injection devices within the respective nest based on analysis of the mass reading information.

In some non-limiting embodiments or aspects, one or more of the steps of process 700 may be performed (e.g., completely, partially, etc.) by controller system 402 (e.g., one or more devices of controller system 402, etc.). In some non-limiting embodiments or aspects, one or more of the steps of process 700 may be performed (e.g., completely, partially, etc.) by another device or a group of devices separate from or including controller system 402, such as RFID reader 404 (e.g., one or more devices of a system of RFID reader 404, etc.), trigger sensor 406 (e.g., one or more devices of a system of trigger sensor 406, etc.), conveyor system 407 (e.g., PLC 410), local database system 411 and/or external database system 412.

As shown in FIG. 7, at step 702, process 700 optionally includes an initial step of acquiring sensitivity data for an RFID tag on each syringe barrel to be retained within a nest. For example, an initial conveyor line 602 may be operated to convey RFID-tagged syringe barrels 102 to a sensitivity testing location where a testing device 604 (i.e., an RFID reader and associated coupling element 606) is operated to measure a sensitivity of the RFID tag 156 of each respective syringe barrel 102. In some embodiments, the testing device 604 also reads the RFID tag 156 of each syringe barrel 102 to retrieve the UDI therefrom, such that the UDI and sensitivity reading may be associated with each other for the particular RFID-tagged syringe barrel 102. Upon acquisition of the sensitivity data and UDI data, step 702, may further including transmitting the acquired data from testing device 604 to system 400 (i.e., to controller system 402, PLC 410, and/or database(s) 411, 412), for inclusion of such data/readings with data/readings acquired during a mass reading performed by system 400.

As shown in FIG. 7, at step 704, process 700 also optionally includes packaging RFID-tagged syringe barrels into a nest (and tub). For example, upon testing of the RFID tag sensitivity (and retrieval of the tag UDI) for each RFID-tagged syringe barrel 102, the syringe barrel 102 is conveyed further down the conveyor line 602 to a transfer device 608 that functions to transfer the syringe barrel 102 to a nest 204, for preparation of a nest 204 of syringe barrels 102 being further packaged (i.e., within tub 202) and then sent to system 400 for further processing.

As shown in FIG. 7, at step 706, process 700 includes controlling another conveyor to start a production line of packages - i.e., of tubs/nests and syringe barrels retained therein. For example, controller system 402 may control conveyor system 407 to cause conveyor 408 to start conveyor movement to move the tubs 202/nests 204 in the production line adjacent to and past antennas 405a, 405b of RFID reader 404, i.e., to the reading location. As an example, controller system 402 (e.g., middleware of controller system 402, etc.) may send a signal to conveyor system 407 (e.g., the PLC 410 of conveyor system 407, etc.) to cause conveyor system 407 to control conveyor 408 to start conveyor movement. In such an example, controller system 402 (e.g., middleware of controller system 402, etc.) may send a signal to arm the RFID reader 404.

As shown in FIG. 7, at step 708, process 700 includes detecting a tub in a predetermined location relative to an antenna of a RFID reader. For example, for each tub/nest of the plurality of tubs 202/nests 204 in the production line, trigger sensor 406 may detect when that tub 202/nest 204 is in a predetermined location relative to antennas 405a, 405b of RFID reader 404 (i.e., at the reading location).

As shown in FIG. 7, at step 710, process 700 includes transmitting a trigger signal to a RFID reader. For example, for each tub/nest of the plurality of tubs 202/nests 204 in the production line, trigger sensor 406 may, in response to detecting that the tub 202/nest 204 is at a predetermined location relative to antennas 405a, 405b of RFID reader 404 in the production line (i.e., at the reading location), transmit a trigger signal to RFID reader 404 to trigger or cause RFID reader 404 to perform a mass reading of the RFID tags 156 of all syringe barrels 102, as well a reading of the nest RFID tag 224 of the nest 204.

As shown in FIG. 7, at step 712, process 700 includes performing a mass reading of the RFID tags of all syringe barrels, as well a reading of the nest RFID tag of a nest. For example, for each tub/nest of the plurality of tubs 202/nests 204 in the production line, RFID reader 404 may, in response to receiving the trigger signal from trigger sensor 406 (e.g., corresponding to that tub/nest, etc.), perform a mass reading of the RFID tags 156 of all syringe barrels 102 therein, as well as read the nest RFID tag 224 of the nest 204. As an example, RFID reader 404 may transmit radio waves via antennas 405a, 405b (and any other additional antennas, or via only a single antenna, according to the configuration of the RFID reader 404/antenna(s)) to RFID tags 156 and RFID tag 224. According to one aspect of the disclosure, the antennas 405a, 405b are positioned at various locations along the conveyor 408 at the reading location, such as on opposing sides of the conveyor 408. When at the reading location, radio waves from the first antenna 405a and the second antenna 405b will activate the RFID tags 156 and RFID tag 224.

Responsive to receiving the radio waves, the device RFID tags 156 and nest RFID tag 224 may transmit a (backscattered) signal that includes identifiers (i.e., UDIs) associated with syringe barrels 102 and the nest 204, back to the first antenna 405a and the second antenna 405b, to be read by RFID reader 404. Additionally, and as described in detail above, information may also be acquired for each device RFID tag 156 during the mass reading regarding: the average received signal strength indicator (RSSI), the read rate (RR), and the response time (T). In some embodiments, the sensitivity of each device RFID tag 156 (acquired at step 702) may be combined with the above information for the device RFID tags 156.

As part of step 712, the RFID reader 404 may transmit or stream, to controller system 402 (e.g., to middleware of controller system 402, etc.) the information or data read from the device RFID tags 156 (e.g., the UDIs, RSSI, RR, T, etc.) and the nest RFID tag 224 (e.g., the UDI).

As shown in FIG.7, at step 714, process 700 includes providing data/information for the RFID-tagged syringe barrels (acquired for the mass reading and, optionally, the sensitivity testing) into an ML model 416, in order to determine the locations of the plurality of RFID-tagged syringe barrels within a nest. That is, for each package 200 that is brought proximate to RFID reader for reading, and with the device RFID tags 156 and nest RFID tag 224 having been read by RFID reader 404 as part of a mass reading, controller system 402 (e.g., middleware of controller system 402, etc.) may immediately input the data/information for the RFID-tagged syringe barrels 102 into an ML model 416, which may be stored in controller system 402, local database system 411 or external database system 412, as non-limiting examples.

As explained in detail above, a supervised learning/training of the ML model 416 is performed in order to enable the model to predict the position of each RFID-tagged syringe barrel 102 within a nest 204 upon the mass reading of the RFID tags 156 of syringe barrels 102. The trained ML model 416 includes a large dataset N of previously analyzed nests (e.g., N > 1000) having RFID-tagged syringe barrels retained therein - with read-related parameters (e.g., RSSI, RR, and T) and optional tag sensitivity provided for the RFID-tagged syringe barrels and the positions (chimney row/seat or line/column) of the RFID-tagged syringe barrels within the nest being known. These input parameters are provided/known for mass readings performed with the RFID reader (and antenna(s) thereof) in a plurality of configurations. Accordingly, the ML model 416 is trained to associate the position of each RFID-tagged syringe barrel within the nest with the read-related parameters for each RFID-tagged syringe barrel.

As shown in FIG. 7, at step 716, process 700 continues with the ML model 416 determining/predicting the position of each RFID-tagged syringe barrel in a given nest. That is, with the ML model 416 trained as described above and the data/information acquired from the mass reading of the RFID-tagged syringe barrels 102 within the nest 204 (at the reading location), along with the optional sensitivity data, the ML model 416 is able to determine the position of each RFID-tagged syringe barrel 102 in the nest 204. With the configuration k of the RFID reader 404 known, the ML model 416 analyzes the information or data read from the device RFID tags 156 (e.g., the UDIs, RSSI, RR, T, sensitivity, etc.) and outputs a register of the positioning of each RFID-tagged syringe barrels 102 within the nest 204, e.g., syringe barrel_1 is in position (x₁;y₁).... syringe barrel_n is in position (xₙ;yₙ).

As shown in FIG. 7, at step 718, process 700 continues with associating the listing/positioning of RFID-tagged syringe barrels 102 to the particular nest 204 - with the UDI of the nest RFID tag 224 of nest 204 associated with the UDIs of the RFID tags 156 of the syringe barrels 102. Accordingly, an aggregated list/register can be generated and stored, such as in local database system 411 or shift register 414 of PLC 410, as non-limiting examples. In some embodiments, the aggregated list/register - including the UDIs of the RFID-tagged syringe barrels 102 retained within a respective nest 204 and a location of each of the plurality of RFID-tagged syringe barrels 102 within the respective nest 204 - is periodically synchronized with external database system 412, such that the aggregated list/register may be accessed by other customers or off-site users.

Beneficially, embodiments of the invention thus provide a system and method for localizing individual RFID-tagged syringe barrels within a nest, such as may be desired when providing packaged syringe barrels (i.e., packaged in a tub and nest) to a customer for performing of a fill and finish process. The system and method may perform a mass reading of RFID-tagged syringe barrels within a nest to acquire UDIs of the syringe barrels, along with a plurality of additional tag-related parameters/values, i.e., read-related parameters. The acquired tag information in input into an ML model 416 that is trained to predict/determine the location of each RFID-tagged syringe barrels within the nest, with the predicted/determined locations of the RFID-tagged syringe barrels aggregated with the nest data, in order to provide detailed information on each package (nest/tub and syringe barrels therein) that is processed by/through the system. The RFID-tagged syringe barrels and location thereof within a nest may thus be registered, thus reducing or eliminating potential registration errors on a downstream fill and finish manufacturing line.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A system for localizing RFID-tagged medical injection devices in a nest, the system comprising:
a conveyor system configured to convey a plurality of nests along a conveying path, each of the plurality of nests having a nest RFID tag thereon, the nest retaining a plurality of RFID-tagged medical injection devices therein each having a device RFID tag;
one or more RFID coupling elements positioned at a reading location along the conveying path;
a RFID reader operably connected with the one or more RFID coupling elements and configured to perform a mass reading of the device RFID tags and a reading of the nest RFID tag upon a respective nest passing the reading location, wherein reading the nest RFID tag comprises reading a unique device identifier (UDI) from the nest RFID tag and reading the device RFID tags comprises reading a UDI for each of the plurality of RFID-tagged medical injection devices, along with read-related parameters of the device RFID tags; and
at least one processor coupled to a memory and comprising a machine learning (ML) model therein, with the ML model configured to:
receive a model input from the RFID reader comprising the UDIs of the device RFID tags and the read-related parameters of the device RFID tags; and
determine a location of each of the plurality of RFID-tagged medical injection devices within the respective nest based on analysis of the read-related parameters of the device RFID tags by the ML model, with the location of each of the plurality of RFID-tagged medical injection devices within the respective nest associated with the UDI for that RFID-tagged medical injection device.

2. The system of claim 1, wherein the read-related parameters of each device RFID tag comprise:
a received signal strength indicator (RSSI) corresponding to a power level of the RFID tag's backscattered response signal;
a read rate (RR) of the device RFID tag; and
a response time (T) of the device RFID tag comprising an amount of time needed for the device RFID tag to respond the first time.

3. The system of claim 2, wherein the system further comprises:
a secondary conveyor configured to individually convey the plurality of RFID-tagged medical injection devices along a secondary conveying path, prior to positioning of the plurality of RFID-tagged medical injection devices in a respective nest; and
a sensitivity testing device configured to perform a sensitivity testing of the device RFID tag of each of the plurality of RFID-tagged medical injection devices upon a respective RFID-tagged medical injection device passing a testing location along the secondary conveyor, to determine a tag sensitivity of the device RFID tag, which is a minimum power needed to read the device RFID tag at a given frequency.

4. The system of claim 3, wherein the ML model is configured to:
receive another model input from the sensitivity testing device comprising the tag sensitivity of the device RFID tags; and
determine the location of each of the plurality of RFID-tagged medical injection devices within the respective nest based on analysis of the read-related parameters and tag sensitivity of the device RFID tags by the ML model, with the location of each of the plurality of RFID-tagged medical injection devices within the respective nest associated with the UDI for that RFID-tagged medical injection device.

5. The system of any of claims 1-4, wherein the ML model is configured to analyze the model input in view of a number of nest samples with sample RFID-tagged medical injection devices stored therein, via which the ML model is trained; and
wherein, for each nest sample, a position of each of the sample RFID-tagged medical injection devices within the nest is known and is associated with read-related parameters of a device RFID tag on each of the sample RFID-tagged medical injection devices.

6. The system of claim 5, wherein each nest sample has a configuration associated therewith, the configuration comprising an arrangement of one or more antennas of a sample RFID reader used to acquire the read-related parameters of the device RFID tag on each of the sample RFID-tagged medical injection devices.

7. The system of claim 6, wherein the ML model is trained with four or more configurations for the nest samples.

8. The system of any of claims 5-7, wherein the number of nest samples is 1,000 or more.

9. The system of any of claims 1-8, wherein the at least one processor is programmed to:
receive from the RFID reader, the UDI of the nest RFID tag; and
associate the UDI of the nest RFID tag with the UDIs of the device RFID tags, so as to provide an aggregated dataset comprising the UDI of the nest RFID tag, the UDIs of the device RFID tags, and the location of each of the plurality of RFID-tagged medical injection devices within the nest.

10. The system of any of claims 1-9, wherein each nest comprises a support plate and a plurality of chimneys extending upwardly from the support plate, each of the plurality of chimneys configured to retain a RFID-tagged medical injection device therein, the plurality of chimneys arranged in a plurality of columns, with each of the plurality of columns comprising a plurality of seats.

11. The system of claim 10, wherein in determining the location of each of the plurality of RFID-tagged medical injection devices, the ML model is configured to register the column and seat of each of the plurality of RFID-tagged medical injection devices.

12. The system of any of claims 1-11, wherein the one or more RFID coupling elements comprises at least a first RFID coupling element and a second RFID coupling element.

13. The system of any of claims 1-12, further comprising at least one trigger sensor configured to detect when a respective nest is at the reading location, and wherein for each nest on the conveyor system, the at least one sensor is configured to transmit a trigger signal to the RFID reader to cause the RFID reader to perform the mass reading of the device RFID tags and read the nest RFID tag in response to detecting that the nest is at the reading location.

14. The system of any of claims 1-13, wherein the plurality of RFID-tagged medical injection devices comprise a plurality of syringe barrels.

15. The system of any of claims 1-14, wherein each of the plurality of nests is retained within a respective tub, with the tubs being conveyed along the conveying path.

16. A method for localizing RFID-tagged medical injection devices in a nest, the method comprising:
conveying each of a plurality of nests, via a conveyor system, along a conveying path, each of the plurality of nests having a nest RFID tag thereon, the nest retaining a plurality of RFID-tagged medical injection devices therein each having a device RFID tag;
providing a RFID reading system at a position along the conveying path, the RFID reading system comprising:
one or more RFID coupling elements positioned at a reading location along the conveying path; and
a RFID reader operably connected with the one or more RFID coupling elements;
performing a mass reading of the device RFID tag on each of the plurality of RFID-tagged medical injection devices via the RFID reading system within a respective nest, as the respective nest passes the reading location, with the mass reading retrieving a unique device identifier (UDI) for each of the device RFID tags, along with read-related parameters of the device RFID tags;
performing a reading of the nest RFID tag of a respective nest via the RFID reading system, as the respective nest passes the reading location, to retrieve a nest UDI from the nest RFID tag; and
based on the reading of each of the device RFID tags, determining, via an artificial intelligence (AI) model provided on at least one processor, a location of each of the plurality of RFID-tagged medical injection devices within their respective nest;
wherein, in determining the location of each of the plurality of RFID-tagged medical injection devices, the method comprises:
receiving a model input from the RFID reader comprising the UDIs of the device RFID tags and the read-related parameters of the device RFID tags; and
determining the location of each of the plurality of RFID-tagged medical injection devices within the respective nest based on analysis of the read-related parameters of the device RFID tags by the ML model, with the location of each of the plurality of RFID-tagged medical injection devices within the respective nest associated with the UDI for that RFID-tagged medical injection device.

17. The method of claim 16, wherein the read-related parameters of each device RFID tag comprise:
a received signal strength indicator (RSSI) corresponding to a power level of the RFID tag's backscattered response signal;
a read rate (RR) of the device RFID tag; and
a response time (T) of the device RFID tag comprising an amount of time needed for the device RFID tag to respond the first time.

18. The method of claim 17, further comprising:
performing a sensitivity testing of the device RFID tag of each of the plurality of RFID-tagged medical injection devices, prior to the mass reading, and via a sensitivity testing device, to determine a tag sensitivity of the device RFID tag, which is a minimum power needed to read the device RFID tag at a given frequency; and
in determining the location of each of the plurality of RFID-tagged medical injection devices:
receiving another model input from the sensitivity testing device comprising the tag sensitivity of the device RFID tags; and
determining the location of each of the plurality of RFID-tagged medical injection devices within the respective nest based on analysis of the read-related parameters and tag sensitivity of the device RFID tags by the ML model, with the location of each of the plurality of RFID-tagged medical injection devices within the respective nest associated with the UDI for that RFID-tagged medical injection device.

19. The method of any of claims 16-18, further comprising causing the ML model to analyze the model input in view of a number of nest samples with sample RFID-tagged medical injection devices stored therein, via which the ML model is trained; and
wherein, for each nest sample, a position of each of the sample RFID-tagged medical injection devices within the nest is known and is associated with read-related parameters of a device RFID tag on each of the sample RFID-tagged medical injection devices.

20. The method of any of claims claim 16-19, wherein each nest comprises a plurality of chimneys each configured to retain a RFID-tagged medical injection device therein, the plurality of chimneys arranged in a plurality of columns, with each of the plurality of columns comprising a plurality of seats, and wherein determining the location of each of the plurality of RFID-tagged medical injection devices comprises registering the column and seat of each of the plurality of RFID-tagged medical injection devices.
